Europäisches Patentamt

European Patent Office

Office européen des brevets

(11) Veröffentlichungsnummer: **0 275 392**

**A1**

## (12) EUROPÄISCHE PATENTANMELDUNG

(21) Anmeldenummer: **87116767.2**

(22) Anmeldetag: **13.11.87**

(51) Int. Cl.⁴ **A61B 17/16**

(30) Priorität: **08.01.87 DE 3700487**

(43) Veröffentlichungstag der Anmeldung:
**27.07.88 Patentblatt 88/30**

(84) Benannte Vertragsstaaten:
**AT BE CH DE ES FR GB GR IT LI LU NL SE**

(71) Anmelder: **List, Heinz-Jürgen**
**Lindenstrasse 56**
**D-7990 Friedrichshafen 1(DE)**

(72) Erfinder: **Richter, Ulrich**
**Ludolfinger Strasse 14**
**D-8500 Nürnberg 30(DE)**

(74) Vertreter: **Göbel, Matthias, Dipl.-Ing.**
**Pruppacher Hauptstrasse 5-7**
**D-8501 Pyrbaum-Pruppach(DE)**

(54) **Chirurgische Knochenbohrmaschine.**

(57) Bei einer chirurgischen Knochenbohrmaschine mit einem im Maschinengehäuse angeordneten elektrischen Antriebsmotor für die Spannvorrichtung der Bohrwerkzeuge und am Maschinengehäuse angeordneten Betätigungsgliedern für elektrische Schaltmittel für den Antriebsmotor, sind zum einfachen und sicheren Schalten von Drehrichtungsänderungen und Drehzahländerungen des Antriebsmotors zwei nebeneinander ausgebildete unabhängige Betätigungsglieder (5, 6) vorgesehen, von denen eines (5) die elektrischen Schaltmittel für eine Drehrichtung und Drehzahländerungen des Antriebsmotors zwischen einem Niedrigstwert und einem Höchstwert und das andere (6) die elektrischen Schaltmittel für die andere Drehrichtung und Drehzahländerungen des Antriebsmotors zwischen einem Niedrigstwert und einem Höchstwert betätigt und zwischen den beiden Betätigungsgliedern (5, 6) ist ein axial frei verschiebliches Sperrglied (19) im Maschinengehäuse angeordnet, das durch selbsttätiges Einschieben in Rastausnehmungen (17, 18) von jeweils einem der beiden Betätigungsglieder (5, 6), diese an einem gleichzeitigen Betätigen der elektrischen Schaltmittel hindert.

Fig. 2

# Chirurgische Knochenbohrmaschine

Die Erfindung betrifft eine chirurgische Knochenbohrmaschine mit einem im Maschinengehäuse angeordneten elektrischen Antriebsmotor für die Spannvorrichtung der Bohrwerkzeuge und am Maschinengehäuse angeordneten Betätigungsgliedern für elektrische Schaltmittel für den Antriebsmotor.

Bei einer bekannten chirurgischen Bohrmaschine (DE-OS 3 317 398) wird vermittels eines im Maschinengehäuse geführten Betätigungsschiebers ein Ein-Ausschalter für den Antriebsmotor und ein im Motorstromkreis liegender veränderlicher Widerstand zu Drehzahländerungen beeinflußt, während Drehrichtungsänderungen des Antriebs motors Abschwenkungen eines unabhängigen Schalthebels erfordern, der einen Kontaktsatz zu Umpolungen betätigt. Abgesehen davon, daß der Kontaktsatz unkontrolliert betätigbar ist, setzen Drehrichtungsänderungen auch ein Umfassen der Bohrmaschine und damit störende Maßnahmen voraus. Außerdem führen der Kontaktsatz und der Schalthebel zu einer aufwendigen Bauweise der bekannten Bohrmaschine.

Die Erfindung hat zur Aufgabe Drehrichtungsänderungen des Antriebsmotors einfacher und sicherer schalten.

Erfindungsgemäß ist diese Aufgabe gelöst durch zwei nebeneinander ausgebildeten unabhängigen Betätigungsgliedern, von denen eines die elektrischen Schaltmittel für eine Drehrichtung und Drehzahländerungen des Antriebsmotors zwischen einem Niedrigstwert und einem Höchstwert und der andere die elektrischen Schaltmittel für die andere Drehrichtung und Drehzahländerungen des Antriebsmotors zwischen einem Niedrigstwert und einem Höchstwert betätigt und daß zwischen den beiden Betätigungsgliedern ein axial frei verschiebliches Sperrglied im Maschinengehäuse angeordnet ist und daß durch selbsttätiges Einschieben des Sperrgliedes in Rastausnehmungen von jeweils einem der beiden Betätigungsglieder, diese an einem gleichzeitigen Betätigen der elektrischen Schaltmittel gehindert sind. Bevorzugt sind die Betätigungsglieder durch Schieber gebildet, die im Abstand übereinander im Griff des Maschinengehäuses angeordnet sind, während als elektrische Schaltmittel Verschiebe-bzw. Drehwiderstände dienen. Mitteln der Betätigungsglieder ist nunmehr möglich beliebig ein drehrichtungsgemäßes Anlaufen und Anhalten bzw. reproduzierbar neues Anlaufen des Antriebsmotors zu bewirken, wobei das verschiebliche Sperrglied jeweils eine mechanische Verriegelung für die Betätigungsglieder zur Vermeidung eines gleichzeitigen Einschiebens beider Betätigungsglieder bildet. Beliebige Drehzahlen bzw. Drehzahlanderungen sind dabei durch mehr oder weniger weites Einschieben der Betätigungsglieder erzielbar. In Ausgestaltung der Bohrmaschine können die Betätigungsglieder durch mit Betätigungsknöpfen versehene Zylinder gebildet sein, die entgegen Federkraft in das Maschinengehäuse einschiebbar sind und ein mit dem jeweils beweglichen Teil der zugeordneten Verschiebe- bzw. Drehwiderstände in Eingriff stehendes Kuppelglied stützen. Zweckmäßig sind die Kuppelglieder auf den Zylindern frei verschieblich angeordnet und mittels der Rückholfedern an Ansätzen der Zylinder gehalten.

Außerdem ist vorgesehen, die Rastausnehmungen an den in Einschieberichtung hinteren Enden durch Schrägflächen und an den in Einschieberichtung vorderen Enden durch Querflächen zu begrenzen. Dies führt zu einem kraftsparenden Verschieben des Sperrgliedes, wobei eine quer zur Einschiebekraft an den Schrägflächen wirkende Komponente die Verschiebebewegungen des Sperrgliedes einfach bewirkt. Vorteilhaft ist das Sperrglied durch einen zylindrischen Stift gebildet. Es besteht aber auch die Möglichkeit zur Verwendung einer Kugel als Sperrglied.

Schließlich ist noch vorgesehen, die beiden Zylinder in ihrer Ruhestellung gemeinsam gleichzeitig freigebbar verrastbar zu machen, um so ein Einschalten zu verhindern. Außerdem wirken die Rückholfedern im Sinne einer Fixierung der Zylinder in der Ruhestellung derselben.

Fernerhin hat sich als vorteilhaft erwiesen, wenn der Antriebsmotor als weitgehend trägheitsarmer Motor ohne Nachlauf ausgebildet ist. Dies wird in einfacher Weise durch die Verwendung selterner Erden als Motorwerkstoff erzielt.

Die Erfindung ist anhand eines Ausführungsbeispiels in der Zeichnung erläutert. Es zeigen:

Fig. 1 eine Knochenbohrmaschine in Seitenansicht,

Fig. 2 ein Teilstück einer Knochenbohrmaschine teilweise im Schnitt,

Fig. 3 ein Betätigungsglied in Draufsicht,

Fig. 4 Teilstücke von Betätigungsgliedern in Seitenansicht, vergrößert,

Fig. 5 Teilstücke von Betätigungsgliedern in Seitenansicht in einer Betätigungsstellung, vergrößert und

Fig. 6 Teilstücke von Betätigungsgliedern in Seitenansicht in der anderen Betätigungsstellung, vergrößert.

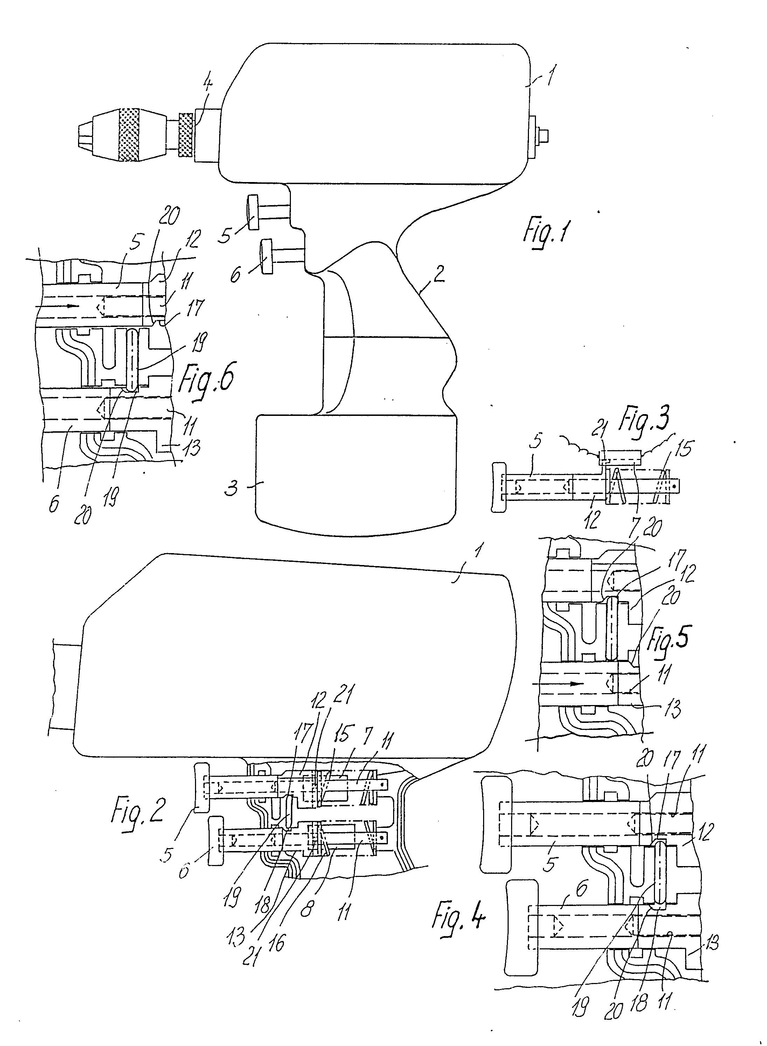

In den Fig. ist mit 1 das Bohrmaschinengehäuse bezeichnet an dem sich ein Griff 2 mit einem Stromversorgungsteil 3 anschließt. Mit 4 ist ein Spannfutter für Bohrwerkzeuge bezeichnet, während 5 und 6 Betätigungsglieder für elektrische Schaltmittel 7 und 8 (Fig. 2) sind, die durch unabhängiges getrenntes Eindrücken jeweils den Antriebsmotor (nicht gezeigt) mit dem Stromversorgungsteil 3 zu Umläufen in der einen bzw. anderen Drehrichtung in Verbindung bringen und im Stromkreis des Antriebsmotors liegende Potentiometer 7 und 8 zu Drehzahländerungen zwischen einem Niedrigst-und Höchstwert betätigen.

Beim Ausführungsbeispiel sind die Betätigungsglieder 5, 6 durch zylindrische Schieber gebildet, die auf gehäusefesten Stiften 11 geführt sind. Die Betätigungsglieder tragen Kuppelglieder 12, 13, die mit Krallen 14 mit dem jeweils beweglichen Teil der Potentiometer 9, 10 in Verbindung stehen. Die Kuppelglieder 12, 13 liegen hierbei an Ansätzen der Zylinder 5,6 durch Rückholfedern 15, 16 an. Die Betätigungsglieder 5, 6 weisen Rastausnehmungen 17, 18 auf, die mit einem frei verschieblichen Sperrstift 19 zur Wirkung bringbar sind. Der Sperrstift 19 ist im Maschinengehäuse gelagert und verhindert als mechanische Sperre das gleichzeitige Einschieben beider Betätigungsglieder und damit unbeabsichtigte Fehlschaltungen.

An dem in Einschieberichtung hinteren Ende sind die Rastausnehmungen 17, 18 durch Schrägflächen 20 begrenzt, während die in Einschieberichtung vorderen Begrenzungsflächen der Rastausnehmungen 17, 18 Querflächen sind.

In der Ruhestellung des Antriebsmotors nehmen die beiden Betätigungsglieder die in Fig. 2 gezeigte äußere Endstellung ein. Die Betätigungsglieder sind durch die Rückholfedern 15, 16 in der äußeren Endstellung (Fig. 4) gehalten. Eine freigebbare Verrastung der Betätigungsglieder in dieser Stellung (nicht gezeigt) ist möglich.

Beim Einschieben des Betätigungsgliedes 5 entgegen der Rückholfeder 15 läuft die Schrägfläche 20 der Rastausnehmung 17 an den Sperrstift 19, wie in Fig. 6 gezeigt, an und verschiebt diesen in die Rastausnehmung 18 des Betätigungsgliedes 6, wobei durch Überlaufen des Sperr stiftes 19 durch das Betätigungsglied 5 der Sperrstift in der Rastausnehmung 18 verbleibt. Das Betätigungsglied 5 wirkt über eine Kralle 21 auf den beweglichen Teil des Potentiometers 7 ein und verschiebt diesen mehr oder weniger weit. Hierbei sind die Verbindung zwischen Stromquelle und Antriebsmotor hergestellt und bestimmte Wertgrößen des Potentiometers 7 zu gewünschten Drehzahlen zwischengeschaltet. Der Antriebsmotor läuft hierbei z.B. in Uhrzeigerdrehrichtung um. Bei Freigabe des Betätigungsgliedes 5 wird dieses durch die Rückholfeder 15 in Richtung Ruhestellung zurückbewegt.

Soll der Antriebsmotor in die andere Drehrichtung umlaufen, so kann dies durch Verschieben des Betätigungsgliedes 6 erreicht werden. Hierbei übt die Schrägfläche 20 der Rastausnehmung 18 eine Verschiebebewegung auf den Sperrstift 19 in Richtung der Rastausnehmung 17 (Fig. 5) aus und das Betätigungsglied 5 ist gegen Einschieben gesperrt. Das Betätigungsglied 6 verschiebt über die Kralle 21 des Kuppelgliedes den beweglichen Teil des Potentiometers 8 . Hierbei sind Umläufe des Antriebsmotors entgegen der Uhrzeigerdrehrichtung mit beliebigen Drehzahlen erzielbar.

Bei Freigeben des Betätigungsgliedes 6 bewirkt die Rückholfeder 16 eine Rückstellbewegung des Betätigungsgliedes 6 in die Stellung der Fig. 2. Die frei verschiebliche Anordnung der Kuppelglieder auf den Betätigungsgliedern 5, 6 ergibt einen Überhub, wodurch bei Rückbewegungen der Betätigungsglieder 5, 6 eine gegebenenfalls vorhandene Trägheit des Potentiometers wirkungslos bleibt.

## Ansprüche

1. Chirurgische Knochenbohrmaschine mit einem im Maschinengehäuse angeordneten elektrischen Antriebsmotor für die Spannvorrichtung der Bohrwerkzeuge und am Maschinengehäuse angeordneten Betätigungsgliedern für elektrische Schaltmittel für den Antriebsmotor, gekennzeichnet durch zwei nebeneinander ausgebildeten unabhängigen Betätigungsgliedern (5, 6), von denen eines (5) die elektrischen Schaltmittel für eine Drehrichtung und Drehzahländerungen des Antriebsmotors zwischen einem Niedrigstwert und einem Höchstwert und das andere (6) die elektrischen Schaltmittel für die andere Drehrichtung und Drehzahländerungen des Antriebsmotors zwischen einem Niedrigstwert und einem Höchstwert betätigt und daß zwischen den beiden Betätigungsgliedern (5, 6) ein axial frei verschiebliches Sperrglied (19) im Maschinenengehäuse angeordnet ist, das durch selbsttätiges Einschieben in Rastausnehmungen (17, 18) von jeweils einem der beiden Betätigungsglieder (5, 6), diese an einem gleichzeitigen Betätigen der elektrischen Schaltmittel gehindert sind.

2. Bohrmaschine nach Anspruch 1, dadurch gekennzeichnet, daß die Betätigungsglieder (5, 6) durch Schieber gebildet sind.

3. Bohrmaschine nach Anspruch 2, dadurch gekennzeichnet, daß die Schieber (5, 6) im Abstand übereinander im Griff (2) des Maschinengehäuses angeordnet sind.

4. Bohrmaschine nach Anspruch 1, dadurch gekennzeichnet, daß die elektrischen Schaltmittel für die Drehzahländerungen durch Verschiebe-bzw. Drehwiderstände (7, 8) gebildet sind.

5. Bohrmaschine nach Anspruch 2 und 3, dadurch gekennzeichnet, daß die beiden Betätigungsglieder (5, 6) durch mit Betätigungsknöpfen versehene Zylinder gebildet und entgegen Federkraft (15, 16) in das Maschinengehäuse einschiebbar sind und daß die Zylinder (5, 6) ein mit dem beweglichen Teil der Verschiebe bzw. Drehwiderstände (7, 8 ) in Eingriff stehendes Kuppelgied (12, 13) stützen.

6. Bohrmaschine nach Anspruch 5, dadurch gekennzeichnet, daß die Kuppelglieder (12, 13) auf den Betätigungsgliedern (5, 6) frei verschieblich angeordnet sind und mittels der Rückholfedern (15, 16) an Absätzen der Betätigungsglieder (5, 6) gehalten sind.

7. Bohrmaschine nach Anspruch 1, dadurch gekennzeichnet, daß die Rastausnehmungen (17, 18) an den in Einschieberichtung hinteren Ende durch Schrägflächen (20) und in Einschieberichtung vorderen Ende durch Querflächen begrenzt sind.

8. Bohrmaschine nach Anspruch 1, dadurch gekennzeichnet, daß das Sperrglied (19) durch einen zylindrischen Stift, eine Kugel od.dgl. gebildet ist.

9. Bohrmaschine nach Anspruch 1, dadurch gekennzeichnet, daß die Betätigungsglieder (5, 6) in der Ruhestellung gemeinsam gleichzeitig freigebbar verrastbar sind.

10. Bohrmaschine nach Anspruch 1, dadurch gekennzeichnet, daß die Betätigungsglieder (5, 6) für die elektrischen Schaltmittel durch eine Wippe gebildet sind.

11. Bohrmaschine nach Anspruch 1, dadurch gekennzeichnet, daß der Antriebsmotor durch die Verwendung selterner Erden als Werkstoff trägheitsarm ist.

0 275 392

Fig.1

Fig.6

Fig.3

Fig.5

Fig.2

Fig.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-2 836 263 (OLYMPIC FISHING TACKLES CO.) <br> * Seite 8, Zeile 25 - Seite 10, Zeile 6; Ansprüche 1, 2; Figuren 1-3 * <br> --- | 1-3,9-11 | A 61 B 17/16 |
| Y | CH-A- 477 870 (SYNTHES AG) <br> * Spalte 2, Zeile 10 - Spalte 3, Zeile 11; Ansprüche, Figur 1 * <br> --- | 1-3,9-11 | |
| A | EP-A-0 167 719 (HOWMEDICA INTERNATIONAL) <br> * Seite 7, Zeile 8 - Seite 8, Zeile 17; Anspruch 1, Figur 1 * <br> --- | 1-3 | |
| A | EP-A-0 078 619 (BLACK & DECKER) <br> * Seite 10, Zeilen 7-33; Anspruch 25; Figur 16 * <br> ----- | 1-3 | |

**RECHERCHIERTE SACHGEBIETE (Int. Cl.4)**

A 61 B 17/00
B 23 B 45/00
B 23 Q 11/00

Der vorliegende Recherchenbericht wurde für alle Patentansprüche erstellt

| Recherchenort | Abschlußdatum der Recherche | Prüfer |
|---|---|---|
| BERLIN | 02-04-1988 | MONNE E.M.B. |